(19) **Europäisches Patentamt**
**European Patent Office**
**Office européen des brevets**

(11) Veröffentlichungsnummer: **0 583 719 A1**

(12) # EUROPÄISCHE PATENTANMELDUNG

(21) Anmeldenummer: **93112789.8**

(22) Anmeldetag: **09.08.93**

(51) Int. Cl.5: **C07C 43/12**, C11D 7/50, C23G 5/028

(30) Priorität: **17.08.92 DE 4227130**

(43) Veröffentlichungstag der Anmeldung:
**23.02.94 Patentblatt 94/08**

(84) Benannte Vertragsstaaten:
**AT BE CH DE DK ES FR GB GR IE IT LI LU NL PT SE**

(71) Anmelder: **Solvay Fluor und Derivate GmbH**
**Hans-Böckler-Allee 20**
**D-30173 Hannover(DE)**

(72) Erfinder: **Buchwald, Hans**
**Am Rodelberg 51 A**
**D-3003 Ronnenberg(DE)**
Erfinder: **Hellmann, Joachim**
**Dohmeyers Weg 25**
**D-3000 Hannover 61(DE)**
Erfinder: **Raszkowski, Boleslaus**
**In der Hespe 246**
**D-3061 Wiedensahl(DE)**

(74) Vertreter: **Lauer, Dieter, Dr.**
**c/o Solvay Deutschland GmbH**
**Hans-Böckler-Allee 20**
**D-30173 Hannover (DE)**

(54) **Zusammensetzungen aus 1-Chlor-2,2,2-trifluorethyldifluormethylether und partiell fluorierten Alkanolen.**

(57) Beschrieben werden neue Zusammensetzungen auf Basis von 1-Chlor-2,2,2-trifluorethyl-difluormethylether im Gemisch mit teilweise fluorierten Alkanolen mit 2 bis 4 C-Atomen, die sich zur Verwendung in Reinigungs- und Trocknungsverfahren sowie als Kühlschmiermittel eignen.

Die vorliegende Erfindung betrifft Zusammensetzungen auf der Basis von 1-Chlor-2,2,2-trifluorethyl-difluormethylether im Gemisch mit partiell fluorierten Alkanolen mit 2 bis 4 C-Atomen, welche geeignet sind zur Verwendung in Verfahren zur Reinigung und Trocknung von Oberflächen sowie zur Verwendung als Kühlschmiermittel.

Es bestand die Aufgabe, neue fluorchlorkohlenwasserstofffreie Zusammensetzungen zur Verfügung zu stellen, die sich als Lösungsmittel in Verfahren zur Reinigung von Oberflächen, in Verfahren zur Wasserentfernung von Oberflächen sowie als Kühlschmiermittel eignen.

Die Aufgabe wird gelöst durch die erfindungsgemäßen Zusammensetzungen, Verwendungen und Verfahren.

Es wurden nun für die genannten Anwendungszwecke geeignete Zusammensetzungen gefunden, die 1-Chlor-2,2,2-trifluor-ethyldifluormethylether und ein partiell fluoriertes Alkanol mit 2 bis 4 C-Atomen oder ein Gemisch dieser partiell fluorierten Alkanole enthalten.

Gegenstand der Erfindung sind daher Zusammensetzungen, die 99,5 bis 50,0 Gew.-% 1-Chlor-2,2,2-trifluorethyl-difluormethylether und 0,5 bis 50,0 Gew.-% eines oder mehrerer teilweise fluorierter Alkanole mit 2 bis 4 C-Atomen enthalten, wobei die Summe der Bestandteile 100 Gew.-% ergibt.

Teilweise fluorierte Alkanole im Sinne der Erfindung sind Alkanole, die außer der OH-Gruppe und den 2 bis 4 Kohlenstoffatomen, Wasserstoff und wenigstens 1 Fluoratom enthalten. Die in den erfindungsgemäßen Zusammensetzungen enthaltenen, teilweise fluorierten Alkanole mit 2 bis 4 C-Atomen sind z. B. Monofluoralkanole wie Monofluorethanole, z. B. 2-Fluorethanol oder Monofluorpropanole, z. B. 3-Fluorpropanol, 1-Fluorpropanol-(2), oder Monofluorbutanole, z. B. 4-Fluorbutanol, oder Difluoralkanole wie Difluorethanole, z. B. 1,1-Difluorethanol, oder Difluorpropanole, z. B. 3,3-Difluorpropanol, 2,2-Difluorpropanol, 1,1-Difluorpropanol-(2), oder Trifluoralkanole wie Trifluorethanole, z. B., 2,2,2-Trifluorethanol, oder Trifluorpropanole, z. B. 3,3,3-Trifluorpropanol, oder Trifluorbutanole, z. B. 4,4,4-Trifluorbutanol, oder Tetrafluoralkanole wie Tetrafluorpropanole, z. B. 2,3,3,3-Tetrafluorpropanol, 2,2,3,3-Tetrafluorpropanol, oder Pentafluoralkanole wie Pentafluorpropanole, z. B. 2,2,3,3,3-Pentafluorpropanol, oder Hexafluoralkanole wie Hexafluorpropanole, z. B. 1,1,1,3,3,3-Hexafluor-2-propanol. Bevorzugt sind dabei als teilweise fluorierte Alkanole die Trifluorethanole, die Tetrafluorpropanole, die Pentafluorpropanole und die Hexafluorpropanole. Besonders bevorzugt sind 2,2,2-Trifluorethanol, 2,2,3,3-Tetrafluorpropanol, 2,2,3,3,3-Pentafluorpropanol und 1,1,1,3,3,3-Hexafluor-2-propanol. In den erfindungsgemäßen Zusammensetzungen können neben 1-Chlor-2,2,2-trifluorethyldifluormethylether die teilweise fluorierten Alkanole entweder einzeln oder auch als Gemisch verschiedener teilweise fluorierter Alkanole vorliegen.

Vorteilhaft sind solche erfindungsgemäßen Zusammensetzungen, die im Bereich von 45 bis 50 °C sieden, wobei binäre Zusammensetzungen aus 1-Chlor-2,2,2-trifluorethyl-difluormethylether und 2,2,3,3-Tetrafluorpropanol, 2,2,3,3,3-Pentafluorpropanol oder 1,1,1,3,3,3-Hexafluor-2-propanol bevorzugt sind. Geeignet ist auch ein binäres Gemisch aus 1-Chlor-2,2,2-trifluorethyldifluormethyletherund 2,2,2-Trifluoretha-nol.

Von den binären Zusammensetzungen sind die binären azeotropartigen bzw. azeotropen Zusammensetzungen besonders bevorzugt. Awendungstechnisch sind diese von großem Vorteil, da sie konstant bzw. im wesentlichen konstant sieden. Damit kommt es nicht zur Fraktionierung der Lösungsmittelbestandteile der erfindungsgemäßen Zusammensetzungen, wodurch unerwünschte Eigenschaftsveränderungen vermieden werden können. Azeotrope bzw. azeotropartige Zusammensetzungen lassen sich nach Gebrauch leicht durch gewöhnliche Destillation reinigen und stehen somit in sehr einfacher Weise für die Wiederverwendung zur Verfügung, ohne daß die Charakteristika der ursprünglichen erfindungsgemäßen Zusammensetzungen dabei verlorengehen.

Insbesondere binäre Zusammensetzungen aus 1-Chlor-2,2,2-trifluorethyl-difluormethylether und 2,2,3,3-Tetrafluorpropanol, 2,2,3,3,3-Pentafluorpropanol oder 1,1,1,3,3,3-Hexafluor-2-propanol mit Siedebereichen von 46 bis 49 °C verhalten sich azeotropartig. Eine Gruppe dieser speziellen azeotropartigen Zusammensetzungen enthält 99,5 bis 97,5 Gew.-% 1-Chlor-2,2,2-trifluorethyl-difluormethylether und 0,5 bis 2,5 Gew.-% 2,2,3,3-Tetrafluorpropanol, wobei diese Zusammensetzungen in einem Siedebereich von 47 bis 49 °C bei Atmosphärendruck sieden. Besonders günstig ist hierbei die azeotrope Zusammensetzung mit etwa 98,7 Gew.-% 1-Chlor-2,2,2-trifluorethyl-difluormethylether und 1,3 Gew.-% 2,2,3,3-Tetrafluorpropanol, die einen Siedepunkt von etwa 47,5 °C bei Atmosphärendruck zeigt. Eine andere Gruppe dieser speziellen azeotropartigen Zusammensetzungen enthält 96,0 bis 93,0 Gew.-% 1-Chlor-2,2,2-trifluorethyl-difluormethylether und 4,0 bis 7,0 Gew.-% 2,2,3,3,3-Pentafluorpropanol, wobei diese Zusammensetzungen in einem Siedebereich von 46 bis 49 °C bei Atmosphärendruck sieden. Besonders günstig ist hierbei die azeotrope Zusammensetzung mit etwa 94,3 Gew.-% 1-Chlor-2,2,2-trifluorethyl-difluormethylether und 5,7 Gew.-% 2,2,3,3,3-Pentafluorpropanol, die einen Siedepunkt von etwa 46,3 °C bei Atmosphärendruck zeigt. Eine weitere Gruppe dieser speziellen azeotropartigen Zusammensetzungen enthält 71,0 bis 66,0 Gew.-% 1-Chlor-2,2,2-trifluorethyl-difluormethylether und 29,0 bis 34,0 Gew.-% 1,1,1,3,3,3-Hexafluor-2-propanol, wobei diese Zusammensetzungen in einem Siedebereich von 46 bis 49 °C bei Atmosphärendruck sieden. Besonders günstig ist hierbei die azeotrope Zusammensetzung mit etwa 68,4 Gew.-% 1-Chlor-2,2,2-trifluorethyl-difluormethylether und 31,6 Gew.-% 1,1,1,3,3,3-Hexafluor-2-propanol, die einen Siedepunkt von etwa 46,1 °C bei Atmosphärendruck zeigt.

Den erfindungsgemäßen Zusammensetzungen können geringe Mengen von an sich bekannten Additiven, z. B. Stabilisatoren, Korrosionsinhibitoren zugesetzt werden. Da sich die obengenannten Gewichtsprozentangaben auf die Summe aus 1-Chlor-2,2,2-trifluorethyl-difluormethylether und teilweise fluoriertem Alkanol beziehen, wird deren relatives Verhältnis durch den Zusatz von Additiven nicht verändert.

Vorzugsweise setzt man als Additive Stabilisatoren den erfindungsgemäßen Zusammensetzungen zu. Bekannte Stabilisatoren sind beispielsweise Nitroalkane, z. B. niedere Nitroalkane, insbesondere Nitroalkane mit 1 bis 4 C-Atomen, wie z. B. Nitromethan oder Nitroethan, Alkylene, z.B. Alkylene mit bis zu 7 C-Atomen, insbesondere Alkylene mit 5 bis 7 C-Atomen, wie z. B. Amylene, Alkylenoxide, z. B. Alkylenoxide mit bis zu 7 C-Atomen, insbesondere Alkylenoxide mit 3 bis 7 C-Atomen, wie z. B.Butylenoxid oder Alkinole, z. B. Alkinole mit bis zu 7 C-Atomen, insbesondere Alkinole mit 3 bis 7 C-Atomen, insbesondere verzweigte Alkinole, wie z. B. 2-Methylbutin-(3)-ol-(2). Diese Stabilisatoren können allein oder miteinander in Kombination zugesetzt werden, wobei Mengen von 0,01 bis 5 Gew.-%, vorzugsweise 0,05 bis 1 Gew.-%, bezogen auf das Gesamtgemisch, gut geeignet sind.

Die erfindungsgemäßen Zusammensetzungen sind bei Raumtemperatur klare Lösungen, die aufgrund ihrer guten anwendungstechnischen Eigenschaften in vielen Anwendungsgebieten eingesetzt werden können.

Ein weiterer Gegenstand der Erfindung ist daher die Verwendung der erfindungsgemäßen Zusammensetzungen zur Reinigung und/oder Trocknung von Oberflächen von Gegenständen. Insbesondere in Tauchund/oder Sprühreinigungsverfahren oder bei Reinigungsverfahren durch Dampfbehandlung von Oberflächen, z. B. der Dampfentfettung, sind die erfindungsgemäßen Zusammensetzungen gut geeignet. Bei diesen an sich bekannten Verfahren wird der zu reinigende Gegenstand in einer oder mehreren Stufen in das flüssige und/oder dampfförmige Reinigungsgemisch getaucht oder mit dem flüssigen Reinigungsgemisch besprüht. Hierbei kann auf an sich bekannte Weise die Reinigungswirkung der erfindungsgemäßen Zusammensetzungen durch geeignete Maßnahmen, z. B. durch die Anwendung bei Siedetemperatur und/oder Anwendung von Ultraschall und/oder Rühren weiter gesteigert werden. Auch durch mechanische Einwirkung, wie z. B. Bürsten kann eine weitere Verbesserung der Reinigungswirkung erreicht werden.

So können die erfindungsgemäßen Zusammensetzungen in der Reinigung und/oder Trocknung von Oberflächen von Gegenständen vorzugsweise zur Reinigung von Kleinteilen aus Metall, Glas, Keramik, Edelsteinen oder aus üblichen Kunststoffen, beispielsweise für die Reinigung von Glaslinsen, Siliciumscheiben, Aluminiumplatten oder Teilen aus Polyethylen verwendet werden. Auch zur Reinigung und/oder Trocknung von elektronischen Bauteilen, Silicium-Wafern, empfindlichen Geräteteilen, Präzisionsgeräten und gedruckten Leiterplatten sind die erfindungsgemäßen Zusammensetzungen geeignet. Vorzugsweise wird die Reinigung von Kleinteilen bzw. Schüttgut wie z. B. Transistor-Kappen in geschlossenen Anlagen, beispielsweise in 1-, 2- bzw. 3-Kammer-Reinigungsanlagen, durchgeführt. Wegen der niedrigen Oberflächenspannung und der guten Benetzungsfähigkeit sind die erfindungsgemäßen Zusammensetzungen dabei auch besonders zur Reinigung von Kapillarsystemen geeignet. Auch zum Strippen von Lack bzw. Lackresten können die erfindungsgemäßen Zusammensetzungen eingesetzt werden.

Bevorzugt eignen die erfindungsgemäßen Zusammensetzungen sich zur Reinigung von Oberflächen von gedruckten Leiterplatten, insbesondere von Leiterplatten, deren Oberflächen mit Lötflußmittelrückständen verunreinigt sind. So lassen sich mit den erfindungsgemäßen Zusammensetzungen die organischen Harzflußmittel, welche in der elektronischen Industrie vorwiegend in Lötverfahren für Leiterplatten eingesetzt werden, problemlos entfernen. Üblicherweise sind die zu entfernenden Harzflußmittel Gemische polarer und nichtpolarer Verbindungen, die oftmals zusätzlich noch an sich bekannte hochaktive, z. B. halogenfreie Aktivatoren enthalten. Überraschenderweise können die erfindungsgemäßen Zusammensetzungen aus 1-Chlor-2,2,2-trifluorethyldifluormethylether und teilweise fluorierten Alkanolen sowohl die polaren als auch die nichtpolaren Komponenten der Harzflußmittel entfernen. Insbesondere zur Entfernung von Lötflußmitteln mit hohem Aktivatorgehalt, sind die erfindungsgemäßen Zusammensetzungen geeignet. So lassen sich Leiterplatten auch bei Verwendung von Flußmitteln mit hohem Aktivatoranteil problemlos mit den erfindungsgemäßen Zusammensetzungen reinigen, ohne daß es hierbei zu unerwünschten "weißen Belägen" kommt, wie sie z. B. bei Verwendung der üblichen Reinigungsmittel beobachtet werden können.

Ferner werden durch die erfindungsgemäßen Zusammensetzungen Gemische zur Verfügung gestellt, die besonders gut für die Trocknung von Gegenständen, d. h., zur Entfernung von Wasser von Oberflächen von Gegenständen geeignet sind. Die zu behandelnden Oberflächen, die feucht oder wassernaß sein können, werden durch die Behandlung mit den erfindungsgemäßen Zusammensetzungen sehr gut von anhaftendem Wasser befreit, d. h. getrocknet. Die Kontaktierung der Oberflächen mit den erfindungsgemäßen Zusammensetzungen kann dabei durch Aufsprühen, Aufbürsten, durch Leiten der Zusammensetzungen über die Oberflächen oder durch Eintauchen der Gegenstände in die Zusammensetzungen erfolgen. Bei Anwendung von Eintauchungstechniken können die Zusammensetzungen dabei in geeigneter Weise bewegt bzw. gerührt oder in anderer Weise in ihrer Wirkung, z. B. durch Ultraschall, unterstützt werden. Die erfindungsgemäßen Zusammensetzungen ermöglichen eine wirksame Trocknung von Gegenständen aus unterschiedlichsten Materialien, beispielsweise von Gegenständen aus Metall, Glas, Keramik, Edelsteinen oder aus Kunststoffen.

Bei der Verwendung der erfindungsgemäßen Zusammensetzungen als Trocknungsmittel bilden diese keine Emulsionen mit Wasser, wodurch sie die erwünschte schnelle Bildung einer getrennten Wasser- und Lösungsmittelphase ermöglichen. Für den Trocknungsgrad ist die Behandlungsdauer nicht besonders kritisch, da eine erhebliche Menge Wasser von den Oberflächen bereits nach anfänglichem Kontakt der Oberflächen mit den erfindungsgemäßen Zusammensetzungen entfernt wird. Üblicherweise werden die zu trocknenden Oberflächen mit der Zusammensetzung etwa 1 Minute lang in Kontakt gebracht, obgleich je nach der Oberflächenbeschaffenheit des Trocknungsgutes bzw. den jeweiligen Umständen des Trocknungsvorganges auch längere oder kürzere Zeiten angewendet werden können. Üblicherweise wird man die Trocknungsverfahren mit den erfindungsgemäßen Zusammensetzungen bei Temperaturen von oberhalb des Erstarrungspunktes von Wasser bis zum Siedepunkt der Zusammensetzung und/oder der Temperatur, bei der sich Wasser immer noch im flüssigen Zustand befindet, durchführen.

Weiterhin betrifft die Erfindung die Verwendung der erfindungsgemäßen Zusammensetzungen als Kühlschmiermittel bei der spanenden, trennenden oder abrasiven Bearbeitung von Metallen. Aufgrund ihrer vorteilhaften physikalischen Eigenschaften wie z. B. der niedrigen Oberflächenspannung, niedrigen Viskosität sowie hohen Dichte sind die erfindungsgemäßen Zusammensetzungen hierfür gut geeignet. Bei ihrer Verwendung als Kühlschmiermittel können die erfindungsgemäßen Zusammensetzungen bei der Bearbeitung von Metallen, beispielsweise beim Bohren, Fräsen, Drehen, Gewindeschneiden, Stanzen, Schneiden oder Schleifen auf alle bekannten Arten angewendet werden. Für diese Anwendungen können insbesondere auch die an sich bekannten Additive auf der Basis von Wachsen oder Wachsderivaten, z. B. die Ester von langkettigen Carbonsäuren und Monoalkoholen mit insgesamt 34 bis 50 C-Atomen, deren sulfochlorierte oder sulfidierte oder durch Hydrierung oder Fluorwasserstoffbehandlung erhaltenen Derivate zugesetzt sein.

Weiterhin können die erfindungsgemäßen Zusammensetzungen als spezielle Lösungs-, Extraktions- und/oder Umkristallisationsmittel in der chemischen und pharmazeutischen Industrie eingesetzt werden.

Die erfindungsgemäßen Zusammensetzungen gewährleisten aufgrund ihrer physikalischen Eigenschaften viele anwendungstechnische Vorteile in den genannten Einsatzgebieten. So zeigen die erfindungsgemäßen Zusammensetzungen überraschenderweise auch ohne weitere polare Zusätze hervorragende Lösungs- bzw. Reinigungseigenschaften. Ein Vorteil der erfindungsgemäßen Zusammensetzungen besteht darin, daß diese aufgrund des hohen Gehaltes an 1-Chlor-2,2,2-trifluorethyl-difluormethylether stark erhöhte und im Falle der azeotropen Zusammensetzungen von 1-Chlor-2,2,2-trifluorethyl-difluormethylether mit 2,2,3,3,3-Pentafluorpropanol oder 1,1,1,3,3,3- Hexafluor-2-propanol sogar keine Flammpunkte mehr aufweisen. Im Gegensatz zu vielen bekannten Lösungsmittelgemischen, die kompliziert zusammengesetzte Vielkomponentensysteme darstellen, bestehen die erfindungsgemäßen Zusammensetzungen nur aus wenigen chemisch weitgehend inerten Bestandteilen. Darüber hinaus erweist es sich als vorteilhaft, daß 1-Chlor-2,2,2-trifluorethyl-difluormethylether gegenüber vollhalogenierten Fluorchlorkohlenwasserstoffen eine leichtere Abbaubarkeit und somit deutlich erhöhte Umweltverträglichkeit zeigt.

Die folgenden Beispiele sollen die Erfindung näher erläutern, ohne sie jedoch in ihrem Umfang zu begrenzen.

## Beispiele

Die Zusammensetzungen wurden durch Zusammenmischen von 1-Chlor-2,2,2-trifluorethyl-difluormethylether mit den entsprechenden teilweise fluorierten Alkanolen in der angegebenen Menge erhalten. Die in den Beispielen angegebenen Angaben in % entsprechen immer Gew.-%.

**Beispiel 1**:

Reinigung von Leiterplatten

In einer handelsüblichen 2- bzw. 3-Kammer-Reinigungsanlage wurden Reinigungsversuche mit Leiterplatten, die sowohl mit üblichen halogenhaltigen Lötflußmitteln als auch mit halogenfreien carbonsäurehaltigen Lötflußmitteln verunreinigt waren, vorgenommen. Sowohl die Verunreinigungen durch übliche halogenhaltige als auch durch halogenfreie Lötflußmittel konnten von den Leiterplatten mit hervorragenden Reinigungsergebnissen entfernt werden. Die Reinigungszusammensetzungen, Reinigungsbedingungen und Reinigungsergebnisse sind in der Tabelle 1 wiedergegeben.

In Tabelle 1 wie in der nachfolgenden Tabelle 2 wird für 1-Chlor-2,2,2-trifluorethy1-difluormethylether die Abkürzung "E" verwendet.

## Tabelle 1

| Nr. Zusammensetzungen für Bad 1 | Reinigungsbedingungen | Ergebnis bei halogen- haltigen Flußmitteln | halogen- freien |
|---|---|---|---|
| 1  E/2,2,2-Trifluor-ethanol: 80,0 % / 20,0 % | 3-Bad-Verfahren: 1) 3 Min. Ultraschall 2) 1 Min. Ultraschall 3) 1 Min. Dampfentfettung (im Bad 2 und 3: E) | ++ | ++ |
| 2  E/2,2,3,3-Tetrafluorpropanol: 98,7 % / 1,3 % | 2-Bad-Verfahren: 1) 3 Min. Ultraschall 2) 1 Min. Dampfentfettung (Zusammensetzung im Bad 2 wie Bad 1) | ++ | + |
| 3  E/2,2,3,3,3-Pentafluorpropanol: 96,0 % / 4,0 % | 2-Bad-Verfahren: 1) 3 Min. Ultraschall 2) 1 Min. Dampfentfettung (Zusammensetzung im Bad 2 wie Bad 1) | ++ | ++ |
| 4  E/2,2,3,3,3-Pentafluorpropanol: 94,3 % / 5,7 % | 2-Bad-Verfahren: 1) 3 Min. Ultraschall 2) 1 Min. Dampfentfettung (Zusammensetzung im Bad 2 wie Bad 1) | ++ | ++ |
| 5  E/2,2,3,3,3-Pentafluorpropanol: 50,0 % / 50,0 % | 3-Bad-Verfahren: 1) 3 Min. Ultraschall 2) 1 Min. Ultraschall 3) 1 Min. Dampfentfettung (im Bad 2 und 3: E) | ++ | ++ |
| 6  E/1,1,1,3,3,3-Hexafluor-2-propanol: 68,4 % / 31,6 % | 2-Bad-Verfahren: 1) 3 Min. Ultraschall 2) 1 Min. Dampfentfettung (Zusammensetzung im Bad 2 wie Bad 1) | ++ | ++ |

E: 1-Chlor-2,2,2-trifluorethyl-difluormethylether

In den in der Spalte "Ergebnis" mit "++" gekennzeichneten Fällen ist eine sehr gute und in den mit "+" gekennzeichneten Fällen eine gute Reinigungswirkung erzielt worden. Es kam nicht zur Bildung "weißer Beläge". Es wird somit deutlich, daß die erfindungsgemäßen Zusammensetzungen hervorragende Reinigungsleistungen zeigen.

**Beispiel 2**:

Reinigung von Schüttgut.

Schüttgut (Transistoren-Kappen) wurden zur Entfernung von Ziehölen in einer 2-Kammer-Anlage (3 Min. Ultraschall, 1 Min. Dampfentfernung) mit einem azeotropen Gemisch aus 94,3 % 1-Chlor-2,2,2-trifluorethyl-difluormethylether und 5,7 % 2,2,3,3,3-Pentafluorpropanol gereinigt. Nach der Behandlung war das Schüttgut einwandfrei sauber.

**Beispiel 3**:

Trocknung von verschiedenen Materialien.

a) In einer 3-Kammer-Trocknungsanlage:

In einer handelsüblichen 3-Kammer-Trocknungsanlage wurden Trocknungsversuche mit Gegenständen aus unterschiedlichen Materialien unter Verwendung eines Gemisches aus 1-Chlor-2,2,2-trifluorethyl-difluormethylether und 2,2,3,3,3-Pentafluorpropanol vorgenommen. So wurden die Trocknungsversuche mit Glaslinsen, Siliciumscheiben, Aluminiumplatten sowie Kunststoffteilen (Polyethylen) durchgeführt. Die 3-Kammer-Trocknungsanlage bestand aus einem Trocknungsbad und zwei hintereinander zum Trocknungsbad kaskardenartig angeordneten Spülbädern, wobei das zweite Spülbad mit einem Überlauf zum ersten Spülbad und das erste Spülbad mit einem Überlauf zum Trocknungsbad versehen war. Über den drei Bädern erstreckte sich ein gemeinsamer Dampfraum, der nach oben zum Zwecke der Lösungsmittelkondensation mit einer Kühlzone ausgestattet war. Das in der Kühlzone abgeschiedene und gesammelte Kondensat wurde über eine Rückführungsleitung in das zweite Spülbad rezirkuliert. Alle drei Kammern der Trocknungsanlage waren zusätzlich mit separaten Heizvorrichtungen ausgestattet. Das Trocknungsbad war durch einen Überlauf mit einer Wasserabscheideeinrichtung verbunden, in der das Trocknungsmittel vom Wasser getrennt wurde. Von der Wasserabscheideeinrichtung wurde das Trocknungsmittel nach der Wasserabtrennung mittels einer Pumpe über eine Rückführungsleitung in das Trocknungsbad rezirkuliert.

Zur Trocknung wurden die Gegenstände zunächst 1. Min. in das Trocknungsbad mit dem siedenden Trocknungsmittel getaucht. Nach der Trocknung im Trocknungsbad wurde der zu trocknende Gegenstand in den zwei Spülbädern mit jeweils siedendem Trocknungsmittel für jeweils 1 Min. unter Eintauchen gespült. Nach einer Verweilzeit von 1 Min. in der Dampfphase verließ der zu trocknende Gegenstand die Anlage absolut trocken und kühl genug, daß man ihn anfassen konnte.

Die Trocknungsmittelzusammensetzung, die Trocknungsbedingungen, die Art der zu trocknenden Gegenstände und die Trocknungsergebnisse sind in der Tabelle 2 (Nr. 1) wiedergegeben.

b) In einer Spritz-Tauch-Dampf-Anlage mit einer Arbeitskammer:

Diese handelsübliche Trocknungsanlage war mit Spritzdüsen, Tauchbad von Raumtemperatur, Siedesumpf, Wasserabscheider, Vorratsbehälter, Umwälzpumpe, Filter und Kühlsystem ausgestattet. In dieser Anlage wurden alle Arbeitsgänge in einer Arbeitskammer, im sogenannten Tauchbad, nur in verschiedenen Arbeitshöhen vorgenommen. Die Dampfphase über dem Tauchbad wurde in dem neben der Arbeitskammer liegenden Siedesumpf erzeugt und gelangte von dort in den Raum über dem Tauchbad.

Die nassen Gegenstände wurden in die Anlage eingeführt und für ca. 30 Sekunden unter vertikaler Bewegung mit einem Trocknungsmittel bei Raumtemperatur knapp über der Oberfläche des Tauchbades abgespritzt. Das Trocknungsmittel bestand aus 94,3 % 1-Chlor-2,2,2-trifluorethyl-difluormethylether und 5,7 % 2,2,3,3,3- Pentafluorpropanol. Das entfernte Wasser gelangte auf die durch Düsen in Bewegung gehaltene Oberfläche des Tauchbades und wurde zusammen mit einer gewissen Menge Trocknungszusammensetzung zum Wasserabscheider abgeflutet, in dem das Wasser vom Trocknungsmittel abgetrennt und nach außen abgeleitet wurde.

Das Trocknungsmittel wurde vom Wasserabscheider in den Vorratsbehälter und von dort zurück in das Tauchbad geleitet.

Zusätzlich wurden in dieser Anlage auch Trocknungsversuche durchgeführt, bei denen die zu trocknenden Gegenstände in das Tauchbad für ca. 1 Min. eingetaucht wurden. Während dieser Verweilzeit wurden sie im Tauchbad bei Raumtemperatur vertikal bewegt. Anschließend wurden die Gegenstände aus dem Trocknungsmittel bis knapp über die Oberfläche des Tauchbades herausgehoben und für ca. 30 Sekunden bei Raumtemperatur mit dem Trocknungsmittel abgespritzt. Nach einer Verweilzeit von ca. 1 Min. in der Dampfphase wurden die Gegenstände der Anlage entnommen.

Auch beim Einsatz der erfindungsgemäßen Zusammensetzung in einer Spritz-Tauch-Dampf-Anlage mit nur einer Arbeitskammer wurden die Gegenstände absolut trocken und fleckenfrei.

Die Trocknungsmittelzusammensetzung, die Trocknungsbedingungen, die Art der zu trocknenden Gegenstände und die Trocknungsergebnisse sind hierfür in der Tabelle 2 (Nr. 2) wiedergegeben.

Tabelle 2

| Nr. | Zusammensetzungen | Trocknungsbedingungen | Material | Ergebnis |
|---|---|---|---|---|
| 1 | Trocknungsbad 1 94,3 Gew.-% E, | 1) 1 Min. siedend getaucht | a) Glaslinsen | absolut trocken |
| | 5,7 Gew.-% 2,2,3,3,3-Pentafluorpropanol | 2) 1 Min. siedend getaucht | b) Siliciumscheiben | absolut trocken |
| | Spülbad 2 und 3 wie Bad 1 | 3) 1 Min. siedend getaucht | c) Aluminiumplatten | absolut trocken |
| | Dampfraum 4 wie Bad 1 | 4) 1 Min. Verweilzeit im Dampfraum | d) Kunststoffteile (Polyethylen) | absolut trocken |
| 2 | Trocknungszusammensetzung in der Arbeitskammer 94,3 Gew.-% E, | 1) $\frac{1}{2}$ Min. gespritzt | a) Glaslinsen | absolut trocken |
| | | 2) 1 Min. getaucht | b) Siliciumscheiben | absolut trocken |
| | 5,7 Gew.-% 2,2,3,3,3-Pentafluorpropanol | 3) $\frac{1}{2}$ Min. gespritzt | c) Aluminiumplatten | absolut trocken |
| | | 4) 1 Min. Verweilzeit im Dampfraum | d) Kunststoffteile (Polyethylen) | absolut trocken |
| E: 1-Chlor-2,2,2-trifluorethyl-difluormethylether | | | | |

**Patentansprüche**

1. Zusammensetzungen, dadurch gekennzeichnet, daß sie 99,5 bis 50,0 Gew.-% 1-Chlor-2,2,2-trifluorethyl-difluormethylether und 0,5 bis 50,0 Gew.-% eines oder mehrerer teilweise fluorierter Alkanole mit 2 bis 4 C-Atomen enthalten, wobei die Summe der Bestandteile 100 Gew.-% ergibt.

2. Zusammensetzungen nach Anspruch 1, dadurch gekennzeichnet, daß sie als teilweise fluorierte Alkanole 2,2,2-Trifluorethanol, 2,2,3,3-Tetrafluorpropanol, 2,2,3,3,3-Pentafluorpropanol und/oder 1,1,1,3,3,3-Hexafluor-2-propanol enthalten.

3. Zusammensetzungen nach Anspruch 2, dadurch gekennzeichnet, daß die Zusammensetzung eine binäre Zusammensetzung aus 1-Chlor-2,2,2-trifluorethyl-difluormethylether und 2,2,2-Trifluorethanol oder 2,2,3,3,-Tetrafluorpropanol oder 2,2,3,3,3-Pentafluorpropanol oder 1,1,1,3,3,3-Hexafluor-2-propanol ist.

4. Zusammensetzungen nach Anspruch 3, dadurch gekennzeichnet, daß die Zusammensetzung eine azeotropartige Mischung aus 99,5 bis 97,5 Gew.-% 1-Chlor-2,2,2-trifluorethyl-difluormethylether und 0,5 bis 2,5 Gew.-% 2,2,3,3-Tetrafluorpropanol enthält, wobei die Summe der Bestandteile 100 Gew.-% ergibt.

5. Zusammensetzungen nach Anspruch 4, dadurch gekennzeichnet, daß die Zusammensetzung eine azeotrope Zusammensetzung aus 98,7 Gew.-% 1-Chlor-2,2,2-trifluorethyl-difluormethylether und 1,3 Gew.-% 2,2,3,3-Tetrafluorpropanol ist.

6. Zusammensetzungen nach Anspruch 3, dadurch gekennzeichnet, daß die Zusammensetzung eine azeotropartige Zusammensetzung aus 96,0 bis 93,0 Gew.-% 1-Chlor-2,2,2-trifluorethyl-difluormethylether und 4,0 bis 7,0 Gew.-% 2,2,3,3,3-Pentafluorpropanol ist, wobei die Summe der Bestandteile 100 Gew.-% ergibt.

7.  Zusammensetzungen nach Anspruch 6, dadurch gekennzeichnet, daß die Zusammensetzung eine azeotrope Zusammensetzung aus 94,3 Gew.-% 1-Chlor-2,2,2-trifluorethyl-difluormethylether und 5,7 Gew.-% 2,2,3,3,3-Pentafluorpropanol ist.

8.  Zusammensetzungen nach Anspruch 3, dadurch gekennzeichnet, daß die Zusammensetzung eine azeotropartige Zusammensetzung aus 71,0 bis 66,0 Gew.-% 1-Chlor-2,2,2-trifluorethyl-difluormethylether und 29,0 bis 34,0 Gew.-% 1,1,1,3,3,3-Hexafluor-2-propanol ist, wobei die Summe der Bestandteile 100 Gew.-% ergibt.

9.  Zusammensetzungen nach Anspruch 8, dadurch gekennzeichnet, daß die Zusammensetzung eine azeotrope Zusammensetzung aus 68,4 Gew.-% 1-Chlor-2,2,2-trifluorethyl-difluormethylether und 31,6 Gew.-% 1,1,1,3,3,3-Hexafluor-2-propanol ist.

10. Zusammensetzungen nach einem der vorhergehenden Ansprüche, dadurch gekennzeichnet, daß die Zusammensetzungen einen Stabilisator in einer Menge von 0,01 bis 5 Gew.-%, vorzugsweise 0,05 bis 1 Gew.-%, bezogen auf das Gesamtgemisch, enthalten.

11. Zusammensetzungen nach Anspruch 10, dadurch gekennzeichnet, daß die Zusammensetzungen einen Stabilisator aus der Gruppe Nitroalkan, Alkylen, Alkylenoxid und/oder Alkinol enthalten.

12. Verwendung der Zusammensetzungen nach Anspruch 1 in Verfahren zur Reinigung und/oder Trocknung von Oberflächen von Gegenständen.

13. Verwendung der Zusammensetzungen nach Anspruch 1 als Kühlschmiermittel.

14. Verfahren zum Reinigen von Oberflächen von Gegenständen, dadurch gekennzeichnet, daß man die Oberflächen mit Zusammensetzungen nach Anspruch 1 behandelt.

15. Verfahren nach Anspruch 14, dadurch gekennzeichnet, daß die zu reinigenden Oberflächen gedruckte Leiterplatten sind.

16. Verfahren zur Wasserentfernung von Oberflächen von Gegenständen, dadurch gekennzeichnet, daß man die Oberflächen mit Zusammensetzungen nach Anspruch 1 behandelt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,P | EP-A-0 525 901 (SOLVAY S.A.) <br> * das ganze Dokument * <br> --- | 1,11-16 | C07C43/12 <br> C11D7/50 <br> C23G5/028 |
| A | US-A-5 023 009 (ABID N. MERCHANT) <br><br> * das ganze Dokument * <br> --- | 1-3,6,7, 11-16 | |
| A | US-A-3 846 332 (LOUISE S. CROIX) <br> * Ansprüche 1-3 * <br> --- | 1 | |
| A | EP-A-0 322 786 (NEW YORK BLOOD CENTER, INC.) <br> * Seite 13; Beispiel 8 * <br> --- | 1 <br> .. | |
| A | US-A-4 999 127 (ABID N. MERCHANT) <br> * Ansprüche 1-9 * <br><br> ----- | 1,12-16 | |
| | | | **RECHERCHIERTE SACHGEBIETE (Int. Cl.5)** <br><br> C07C <br> C11D <br> C23G |

Der vorliegende Recherchenbericht wurde für alle Patentansprüche erstellt

| Recherchenort | Abschlußdatum der Recherche | Prüfer |
|---|---|---|
| BERLIN | 26 NOVEMBER 1993 | RUFET J. |